(19) Europäisches Patentamt
European Patent Office
Office européen des brevets

(11) **EP 4 092 580 A1**

(12) **EUROPEAN PATENT APPLICATION**

(43) Date of publication:
**23.11.2022 Bulletin 2022/47**

(21) Application number: **22173023.7**

(22) Date of filing: **12.05.2022**

(51) International Patent Classification (IPC):
**G06N 3/04** *(2006.01)*        **G06N 3/08** *(2006.01)*
**G06F 3/01** *(2006.01)*

(52) Cooperative Patent Classification (CPC):
**G06N 3/0454; A61B 5/7267; G06F 3/015;
G06N 3/0472; G06N 3/08; G06N 3/082;
G06N 3/088; G06N 20/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.05.2021  US 202163188085 P
17.05.2021  US 202163189527 P**

(71) Applicant: **Teledyne Scientific & Imaging, LLC
Thousand Oaks, CA 91360 (US)**

(72) Inventors:
 • **Simons,, Stephen B.**
  **Raleigh, North Carolina (US)**
 • **Peot,, Mark Alan**
  **Chapel Hill, North Carolina (US)**
 • **Stephens,, Thomas**
  **Durham, North Carolina (US)**
 • **Cafaro,, Jon**
  **Raleigh, North Carolina (US)**
 • **MacRae,, Ryan**
  **Raleigh, North Carolina (US)**

(74) Representative: **K&L Gates LLP
Karolinen Karree
Karlstraße 12
80333 München (DE)**

(54) **METHOD FOR NEURAL SIGNALS STABILIZATION**

(57)    A method for stabilizing disrupted neural signals received by a brain-computer interface (BCI), where a translation model is trained on a clean and disrupted dataset and is used to translate a disrupted signal to a clean signal. The clean dataset is based on the data that is received the same day the BCI is calibrated and the disrupted dataset is based on data received the same day that the model is trained. Based on the variation in daily signal disruption, the training model is retrained each day and a new translation model is applied to a disrupted dataset.

FIG. 1

**Description**

**STATEMENT OF GOVERNMENTAL INTEREST**

The subject matter described in the present disclosure was developed with U.S. Government support under DARPA Intelligent Neural Interfaces program contract number 5T038. The U.S. Government has certain rights in the subject matter.

**CROSS-REFERENCE TO RELATED APPLICATIONS**

This application claims the benefit of priority under 35 U.S.C. § 119(e) to U.S. Provisional Patent Application No. 63/188,085, titled METHOD FOR NEURAL SIGNALS STABILIZATION, filed May 13, 2021, the entire disclosure of which is incorporated by reference herein.

This application claims the benefit of priority under 35 U.S.C. § 119(e) to U.S. Provisional Patent Application No. 63/189,527, titled METHOD FOR NEURAL SIGNALS STABILIZATION, filed May 17, 2021, the entire disclosure of which is incorporated by reference herein.

**TECHNICAL FIELD**

A brain computer interface (BCI) comprises a neural interface that receives neural signals from brain neurons or peripheral nerves. The BCI translates the neural signals into stimulus identities or cognitive/behavioral intents that are mapped to a desired function or neurological muscular command. For example, a BCI may be used in conjunction with a prosthetic device to greatly improve the patient experience and operability of the prosthetic device. In this example, neural signals measured at the brain or periphery are configured to control the prosthetic by mapping them to the user's desired motor intent (e.g. how they want to move their hand). A BCI may also be configured to move a computer cursor or robotic arm for patient with tetraplegia.

Brain implanted BCIs typically use one of two interface types, an electrocorticography (ECoG) interface or a Multi-electrode array (MEA) interface. MEAs are often implanted on the cortical surface of the brain and can measure action potentials, referred to as spikes, from local populations of neurons. The spikes measured on each electrode or assigned to individual neurons, can be decoded by their event frequency in peristimulus time histograms (PSTHs). In contrast to MEAs, ECoG arrays do not measure spikes from individual neurons, but instead measure correlated neural activity from large populations of neurons in local field potentials (LFPs).

Regardless of the interface type, neural signals (e.g. spikes or LFPs) are susceptible to signal degradation from physical or biological changes. The observed spikes measured with MEAs can change for many reasons including: electrode malfunction or movement, and local neuron death or adaptation. These changes in the observed spikes may lead to disruption of the observed stimulus responses including the entire loss of an electrode channel or more subtle differences in the stimulus-specific spike rates. Similarly, the LFPs measured with ECoG can experience changes to their power spectrum. To restore the functionality of the BCI, patients typically need to make frequent visits to a clinician to have the BCI recalibrated for these changes in measured signal. This process of manual recalibration is a problem with chronically implanted neural interfaces and greatly limits the potential of BCIs.

**BACKGROUND**

The need for constant recalibration of a BCI greatly inconveniences both the patient and the clinician/scientist. BCIs function optimally on the day of calibration when the neural signals are clean and most similar to the dataset used to train the BCI. However, each subsequent day of use may increase the level of degradation of the neural signals and may result in the overall decline in the functionality of the BCI. After a short period of time, the neural signals may become distorted to a point where the BCI is unable to process the incoming neural signals at all. The substantial benefits of the BCI implants may be negated by the recalibration schedule required to keep the devices working properly. Further, the calibration process may only be made with knowledge of the BCI interface type and BCI application.

**SUMMARY**

In one aspect, the present disclosure provides a method for translating disrupted neural signals comprising: receiving, at a brain-computer interface (BCI), a first set of neural signals and a second set of neural signals, wherein the first set of neural signals comprises $day_0$ data and wherein the second set of neural signals comprises $day_i$ data; transmitting, to a computing device comprising at least one processor coupled to at least one memory unit, the first set of neural signals and the second set of neural signals and storing the signals in a first datastore and a second datastore of the computing device; training, by the computing device, a plurality of neural translation models in an adversarial networks for neural interfaces (ANNI) method, wherein the ANNI method trains a plurality of translation models, and wherein the plurality of translation models comprise a first autoencoder model, a second autoencoder model, a disrupted recovery model, an artificial disruption model, a first discriminator model, a second discriminator model, shared latent space model, a shared signal space model, and a penalty drift model with training objectives that achieve a shared latent space which retains signal class information, and penalize signal class swapping; generating, by the computing device, a model loss function for each of the plurality of the neural translation models; deriving, by the computing device, a weighting value for each of the plurality of neural translation models, wherein the weighting value corresponds to the loss function for each of the plurality of neural translation models; calculating, by the computing device, an internal metric value for each epoch; determining, by the computing device, whether the internal metric value meets a predetermined value or meet a predetermined number of epochs; updating, by the computing device, the weighting values for the plurality of neural translation models when the predetermined internal metric value is not met; and translating, by the BCI, a plurality of $day_{i+1}$ incoming signals based on the selected model from the $day_i$ signals, wherein today is $day_{i+1}$; and wherein a $day_{i+1}$ translation model is not yet selected; selecting, by the computing device, the $day_i$ translation model based on the weighting values of the plurality of neural translation models that correspond to the epoch with the highest internal metric value; and translating, by the BCI, the $day_{i+1}$ incoming neural signals with the $day_i$ translation model.

In yet another aspect, the present disclosure provides a method for translating disrupted neural signals comprising: receiving, at a brain-computer interface (BCI), a first set of neural signals and a second set of neural signals, wherein the first set of neural signals comprises $day_0$ data and wherein the second set of neural signals comprises $day_i$ data; transmitting, to a computing device comprising at least one processor coupled to at least one memory unit, the first set of neural signals and the second set of neural signals and storing the signals in a first datastore and a second datastore of the computing device; training, by the computing device, a plurality of neural translation models in an adversarial networks for neural interfaces (ANNI) method, wherein the ANNI method trains a plurality of translation models, and wherein the plurality of translation models comprise a first autoencoder model, a second autoencoder model, a disrupted recovery model, an artificial disruption model, a first discriminator model, a second discriminator model, shared latent space model, a shared signal space model, and a penalty drift model with training objectives that achieve a shared latent space which retains signal class information, and penalize signal class swapping; generating, by the computing device, a model loss function for each of the plurality of the neural translation models; deriving, by the computing device, a weighting value for each of the plurality of neural translation models, wherein the weighting value corresponds to the loss function for each of the plurality of neural translation models; calculating, by the computing device, an internal metric value for each epoch; determining, by the computing device, whether the internal metric value meets a predetermined value or meet a predetermined number of epochs; updating, by the computing device, the weighting values for the plurality of neural translation models when the predetermined internal metric value is not met; and translating, by the BCI, a plurality of $day_{i+1}$ incoming signals based on the selected model from the $day_i$ signals, wherein today is $day_{i+1}$; and wherein a $day_{i+1}$ translation model is not yet selected; selecting, by the computing device, the $day_i$ translation model based on the weighting values of the plurality of neural translation models that correspond to the epoch with the highest internal metric value; and translating, by the BCI, the $day_{i+1}$ incoming neural signals with the $day_i$ translation model.

In addition to the foregoing, various other method and/or system and/or program product aspects are set forth and described in the teachings such as text (e.g., claims and/or detailed description) and/or drawings of the present disclosure.

The foregoing is a summary and thus may contain simplifications, generalizations, inclusions, and/or omissions of detail; consequently, those skilled in the art will appreciate that the summary is illustrative only and is NOT intended to be in any way limiting. Other aspects, features, and advantages of the devices and/or processes and/or other subject matter described herein will become apparent in the teachings set forth herein.

In one or more various aspects, related systems include but are not limited to circuitry and/or programming for effecting herein-referenced method aspects; the circuitry and/or programming can be virtually any combination of hardware, software, and/or firmware configured to effect the herein-referenced method aspects depending upon the design choices of the system designer. In addition to the foregoing, various other method and/or system aspects are set forth and described in the teachings such as text (e.g., claims and/or detailed description) and/or drawings of the present disclosure.

The foregoing summary is illustrative only and is not intended to be in any way limiting. In addition to the illustrative aspects and features described above, further aspects and features will become apparent by reference to the drawings and the following detailed description.

## BRIEF DESCRIPTION OF THE DRAWINGS

FIG. 1 illustrates a method for training a neural signal translation model in adversarial networks for neural interfaces (ANNI) method, executed by a computing device, in accordance with at least one aspect of the present disclosure.

FIG. 2 illustrates an individual neural network components used by the ANNI method for training a neural signal translation model, in accordance with at least one aspect of the present disclosure.

FIG. 3 illustrates a process for signal generation by encoders and decoders, in accordance with at least one aspect of the present disclosure.

FIG. 4 illustrates a signal translation process for a disrupted signal to a recovered signal through the recovery pathway, in accordance with at least one aspect of the present disclosure.

FIG. 5 illustrates a signal translation process for a clean signal to a disrupted signal through an artificial disruption pathway, in accordance with at least one aspect of the present disclosure.

FIG. 6 illustrates a discriminator portion of the ANNI method shown in Fig. 2 including $Disc_A$ and $Disc_B$, in accordance with at least one aspect of the present disclosure.

FIG. 7 illustrates a process for selecting and implementing a trained translation model, in accordance with at least one aspect of the present disclosure.

FIG. 8 illustrates a process of using a trained translation model of a prior day as a potential translation model for a subsequent day, in accordance with at least one aspect of the present disclosure.

FIG. 9 illustrates one aspect of a neural signal stabilization system comprising a wearable physiological signal amplifier system that records 32 channels of high-fidelity neural signals with water based electrodes, in accordance with at least one aspect of the present disclosure.

FIG. 10 illustrates an architectural or component view of a computing system 800 that may be employed to implement the ANNI method for training and selecting a neural signal translation model described in connection with FIGS. 1-8, in accordance with at least one aspect of the present disclosure.

## DESCRIPTION

Applicant of the present application owns the following U.S. Patents and Patent Applications, the disclosure of each of which is herein incorporated by reference in its entirety:

- U.S. Patent Application No. 17/160,442, titled SYSTEM AND METHOD OF SLEEP INDUCTION, filed January 28, 2021;
- U.S. Patent Application No. 16/354,029, titled METHOD FOR NON-INVASIVE ENHANCEMENT OF DEEP SLEEP, filed March 14, 2019, now abandoned;
- U.S. Patent Application No. 15/720,621, titled APPARATUS, SYSTEM, AND METHODS FOR TARGETED MEMORY ENHANCEMENT DURING SLEEP, filed September 29, 2017, now U.S. Patent No. 10,660,569; and
- U.S. Patent Application No. 15/678,848, titled SYSTEM AND METHOD FOR NONINVASIVE IDENTIFICATION OF COGNITIVE AND BEHAVIORAL GOALS, filed August 16, 2017, now U.S. Patent No. 10,945,864.

In one aspect, the present disclosure is directed to an unsupervised and automated method for stabilizing neural signals received by a brain-computer interface (BCI). The method may be used on a variety of signal types and therefore is agnostic to the type of interface, (e.g. electrocorticography (ECoG) or Multi-electrode arrays (MEAs)), and the type of signal degradation. The method employs a neural network to train a plurality of models based on a clean Dataset A and a disrupted Dataset B. In the present aspect, the disrupted Dataset B refers to a representative set of signals captured at any time point beyond the initial point of BCI calibration/training. The trained neural network model is configured to translate the signals from the disrupted Dataset B to their analogous exemplars from the clean Dataset A. The training method is based on a cycle consistent generative adversarial network (GAN). This architecture is specifically implemented for a network analogous to the unsupervised image to image translation model (UNIT) that uses unpaired input samples. The method for translating and stabilizing neural signals may be applied to an existing BCI as a modular application or integrated into a new BCI. Due to the variability of signal disruption factors, a plurality of models are trained providing multiple opportunities to develop a model that produces a high translation accuracy for the observed signal disruption. Once the plurality of models are trained, the accuracy of each model is predicted using a set of internal metrics without supervision. The model with the highest predicted accuracy is selected and applied to translate new signals measured at the neural interface at future time points. Each subsequent day, the incoming neural signals may experience further degradation and require the models to be retrained and reselected each day. In various aspects, the selected model from the previous day may be used as one of the plurality of models that is trained for the subsequent day.

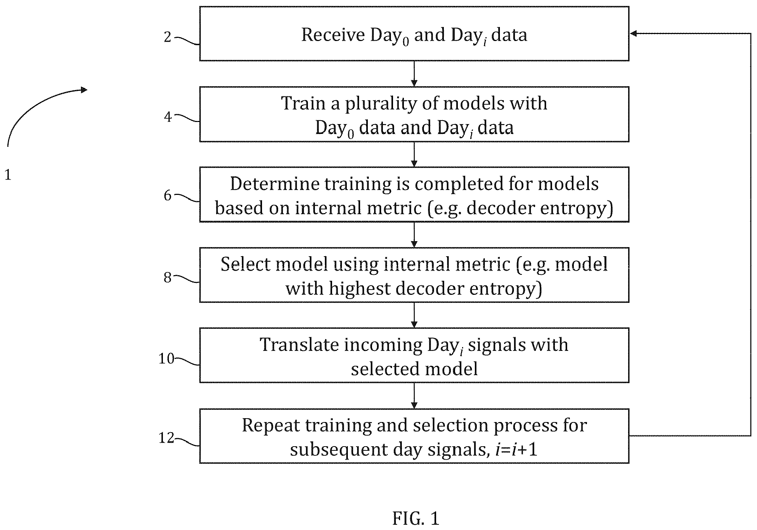

FIG. 1 illustrates a method 1 for training a neural signal translation model in adversarial networks for neural interfaces (ANNI) method, executed by a computing device, in accordance with at least one aspect of the present disclosure. In various aspects, the method 1 may be executed by a computing device internal or external to the BCI, as described hereinbelow. In accordance with the method 1, a BCI receives 2 reference data for a training algorithm including

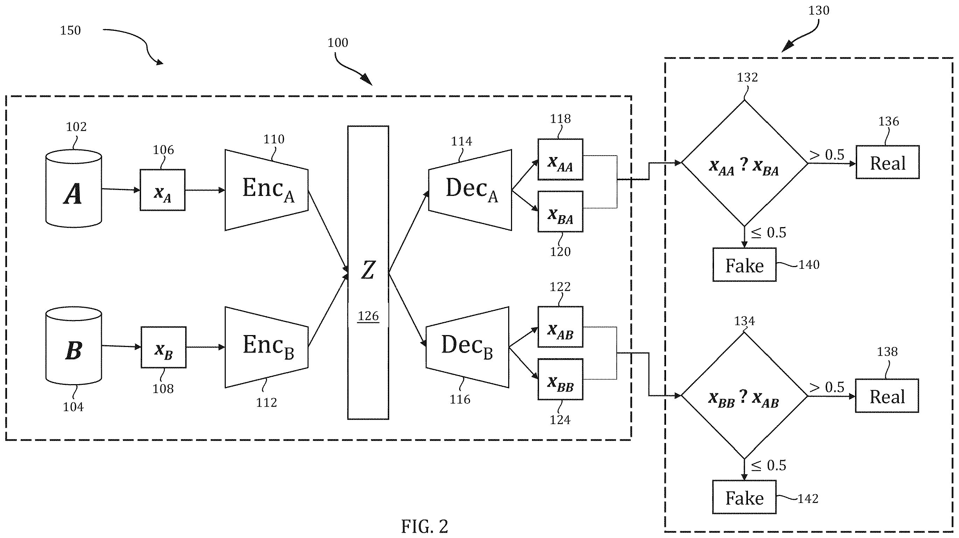

$day_0$ and $day_i$ data. In the context of the present disclosure, $day_0$ data is a reference dataset of clean neural signals and $day_i$ data is a reference dataset of disrupted neural signals, where i is a positive integer greater than 1. The ANNI method concurrently trains a clean translation model on $day_0$ and a disruption translation model on $day_i$ data to optimize the accuracy of the underlying model. The $day_0$ data is recorded the same day that the BCI is calibrated or recalibrated, and is used as the target translation data. The $day_0$ signals are considered to be the most accurate representation of the behavioral intent and are stored in Dataset A as the clean $day_0$ dataset. The subsequently measured neural signals, recorded on $day_i$, are stored as the disrupted signal dataset, Dataset B. The ANNI method trains 4 a plurality of models to translate or recover the signals in of Dataset B back to their original state represented in Dataset A. The accuracy of trained models may be determined by any number of internal metrics that are intrinsic to Dataset A and/or a trained neural decoder present in the BCI. In one aspect, an internal metric may compare the recovered signal distributions ($X_{BA}$ in FIG. 2) according to the mean and variance of the signals in Dataset A, and selecting the point in training with the closest match. If a neural decoder is present and accessible, one can also use the distribution of expected decoded values (e.g. decoder entropy). The ANNI method determines 6 that the trained model meets a predetermined accuracy threshold, where the model is deemed to be maximally accurate based on decoder entropy, or once a training model has achieved an optimal value for the selected internal metric in a predetermined amount of time. Each internal metric value of each completely trained model is compared by the computing device to determine the most accurate or best performing model for that day. The computing device selects 8 the translation model based on the model with the highest decoder entropy or internal metric value. The BCI translates 10 the incoming signals for $day_{i+1}$ with the selected model and the selected model may be retrained according to the disrupted signals each day. Accordingly, the ANNI method repeats 12 the training and selection process for subsequent day signals, $i = i+1$.

FIG. 2 illustrates an individual neural network components used by the ANNI method 150 for training a neural signal translation model, in accordance with at least one aspect of the present disclosure. The training process employs a variation of a GAN architecture 150, herein referred as ANNI, e.g., adversarial networks for neural interfaces. A plurality of neural brain signal model segments are trained in the ANNI method 150 to translate incoming disrupted neural signals to recovered neural signals. Generally, the ANNI method 150 comprises a generator 100 and a discriminator portion 130 configured to receive signals from the generator 100 and process the received signals in accordance with methods described herein.

Turning first to description of the generator 100 of the ANNI architecture 150, the generator 100 comprises two encoders 110, 112, a shared latent space 126, and two decoders 114, 116. The output of the generator 100 is dependent on the translation path, where different translation paths correspond to different model layers. Depending on the signal translation path, different model layers may be applied to the signal in the encoding, mapping, or decoding phase. The generator outputs $x_{AA}$ signals 118 and $x_{BB}$ signals 120 from the autoencoder pathways, $x_{AB}$ signals 122 from the artificial disruption pathways, and $x_{BA}$ signals 120 from the recovery pathways. The encoders 110, 112 and decoders 114, 116 may use a plurality of different neural signal simulation models that correspond to MEA or ECoG interfaces and different biological or physical signal disruptions.

Description of Generator Architecture

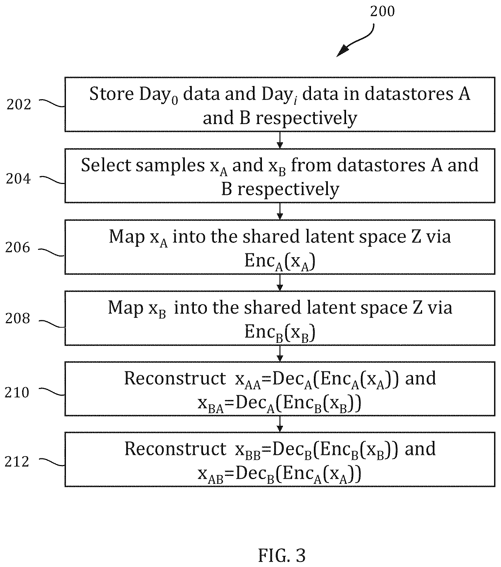

The model segments are implemented in various layers of the encoders and decoders in the generator of the ANNI, and FIG. 3 illustrates one aspect of a signal generation process 200 for the ANNI method. As discussed, input signals $x_A$ and $x_B$ correspond to signals $day_0$ and $day_i$ respectively and are store in datastores A and B, at 202. Signals $x_{AA}$, $x_{BB}$, $x_{AB}$, and $x_{BA}$, are the output of the generator 100, where the autoencoder pathways produce $x_{AA} = Dec_A(Enc_A(x_A))$ and $x_{BB} = Dec_B(Enc_B(x_B))$, the recovery path produces $x_{BA} = Dec_A(Enc_B(x_B))$, and the artificial disruption path produces $x_{AB} = Dec_B(Enc_A(X_A))$. The signals are sampled 204 from each datastore and are mapped 206, 208 into the shared latent space corresponding to a class-dependent transformation. The signals are reconstructed by decoders $Dec_A$ 210 or $Dec_B$ 212 and are passed to the discriminator portion of the ANNI architecture.

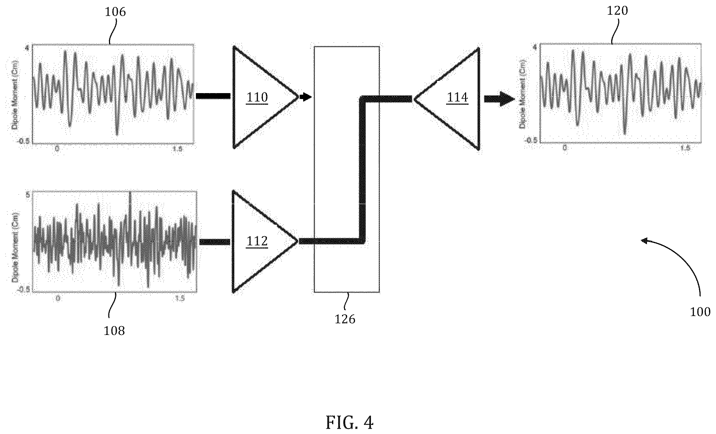

FIG. 4 illustrates the recovery pathway for a disrupted $day_i$ signal. Signal $x_B$ 108 is received from the disrupted signal datastore B, encoded by $Enc_B$ 112, and mapped into the shared latent space z 126. Autoencoder signal $x_A$ 106 is also mapped to the clean domain of shared latent space z 126. Signals $x_A$ 106 and $x_B$ 108 are reconstructed by $Dec_A$ 114, and input singal $x_B$ 108 is outputted as a recovered signal $x_{BA}$ 120.

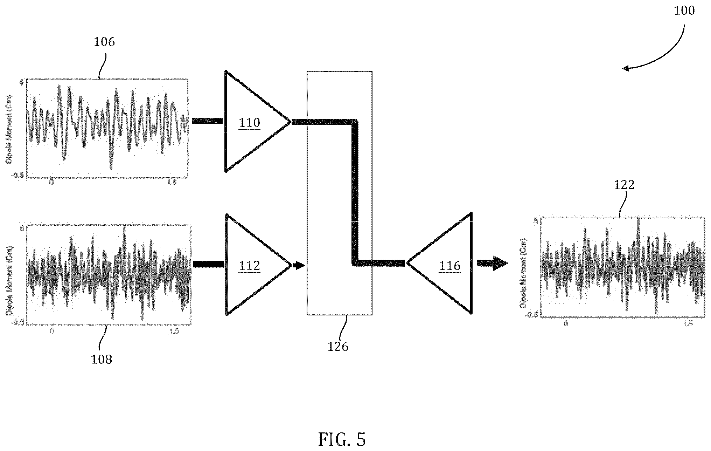

FIG. 5 illustrates the artificial disruption pathway for a clean $day_0$ signal. Signal $x_A$ 106 is received from the clean signal datastore A, encoded by $Enc_A$ 110, and mapped to the disrupted domain of shared latent space z 126. Autoencoder signal $x_B$ 108 is also mapped to the clean domain of shared latent space z 126. Signals $x_A$ 106 and $x_B$ 108 are reconstructed by $Dec_B$ 116, and input singal $x_A$ 106 is outputted as an artificially disrupted signal $x_{AB}$ 122. The same encoding and decoding process is performed with autoencoder signals $x_{AA}$ and $x_{BB}$, where the encoding and decoding is designed produce original signals $x_A$ and $x_B$.

The neural network topology of the encoders and decoders are feed-forward, rectified linear unit (ReLU) networks, with dropout between layers. Neither activation functions nor dropout are applied in the final layer of the encoder in order to maintain a maximally expressive latent space. Additionally, the dropout signal simulation is not applied before the final layer of the decoder in order to enable the greatest fidelity in reconstructed signals. The network topologies are fully-connected for spiking interfaces, when there are no assumptions of spatial correlation in signal space. Convolutional networks can be used for ECoG arrays since spatial correlation in those signal spaces may exist (particularly for µECoG where adjacent electrodes in the grid are separated by small distances).

Description of the Discriminator Architecture

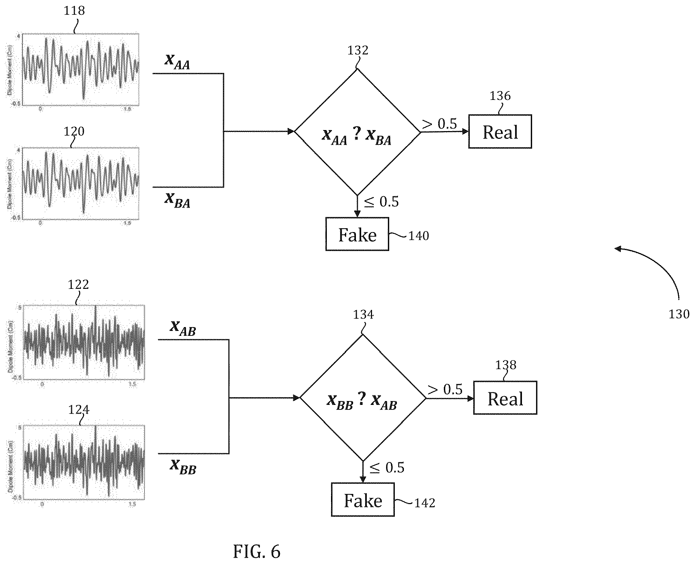

The discriminator is trained to classify incoming signals as "real" or "fake", where real corresponds to having come from the original Datasets A or B, and "fake" corresponds to having been an artificially disrupted or a recovered signal. FIG. 6 illustrates the discriminator portion 130 of the ANNI method, where the signals $x_{AA}$ 118, $x_{BA}$ 120, $x_{AB}$ 122, and $x_{BB}$ 124, are received from the generator. Discriminator $Disc_A$ 132 receives signals $x_{AA}$ 118 and $x_{BA}$ 120 corresponding to the clean domain, and discriminator Discs 134 receives signals $x_{AB}$ 122 and $x_{BB}$ 124 corresponding to the disrupted signal domain. In each domain, a signal is sampled from a datastore and generated according to a translation model to approximate the sampled signals from the datastore. The discriminator attempts to accurately distinguish a sampled signal "real" from a generated signal "fake". $Disc_A$ 132 determines the probability that signals 118 and 120 are a real or fake clean neural signal, and $Disc_B$ 134 determines the probability that signals 122 and 122 are a real or fake disrupted neural signal.

The discriminator architectures are feed-forward rectified linear unit (ReLU) binary classification networks with a sigmoid applied to the output layer. Each discriminator operates in the signal space and outputs a prediction value between 0 and 1, where $Disc_A(x_n) > 0.5$ is interpreted as a "real" signal, or having come from Datastore A, and $Disc_A(x_n) \leq 0.5$ is interpreted as a "fake" signal, or having come through the recovery pathway $Dec_A(Enc_B(x_B))$. Similarly, $Disc_B(x_n) > 0.5$ is interpreted as a "real" signal, or having come from datastore B, and $Disc_{B(x_n)} \leq 0.5$ is interpreted as a "fake" signal, or having come through the artificial disruption pathway $Dec_B(Enc_A(x_A))$.

Loss functions and Weight updates to Training Models

Following a training epoch, the networks in the training model are updated according the discriminator results. The first step in updating the training model weights is to update the discriminator models to more accurately distinguish between incoming signals and generated signals. The discriminator output is evaluated according to a binary cross-entropy (BCE) training objective and produces a discriminator loss function. A loss function is defined for each discriminator $Disc_A$ 132 and $Disc_B$ 134, where

$$L_{Disc}(Disc_A) := BCE(Disc_A(x_{AA}), 1) + BCE(Disc_A(x_{BA}, 0))$$

and

$$L_{Disc}(Disc_B) := BCE(Disc_B(x_{BB}), 1) + BCE(Disc_B(x_{AB}, 0)).$$

Once the discriminator loss functions are evaluated, the generator loss functions may be evaluated for the recovery pathway, $x_{BA} := Dec_A(Enc_B(x_B))$, and the artificial disruption pathway, $x_{AB} := Dec_B(Enc_A(x_A))$. These signals may be considered as part of the "generative" processes since the resulting signal is only statistically related to signals collected from the BCI, and has no counterpart in either dataset. The loss function for these generative signals is determined according to the extent that the generator models were able to fool the discriminators, where

$$L_{Gen}(Gen_{BA}) := BCE(Disc_A(x_{BA}), 1)$$

$$L_{Gen}(Gen_{AB}) := BCE(Disc_B(x_{AB}), 1)$$

Only the weights in the generator pathways are updated at this step. The loss $L_{Gen}(Gen_{BA})$ may be used to update the networks $Enc_B$ and $Dec_A$, while the loss $L_{Gen}(Gen_{BA})$ may be used to update the networks $Enc_A$ and $Dec_B$. The purpose of interpreting the recovery (and artificial disruption) pathway as a generator, and guiding that generator using a discriminator, is to obtain a non-disrupted representation of each incoming neural recording that is statistically similar to the non-disrupted dataset. This does not solve the class matching problem, which is addressed though Cycle Consistency and an ad-hoc drift penalty.

In this stage, an ad-hoc penalty is included on the dynamic range of the generator's output. The discriminators are typically very tolerant of variance in the dynamic range of their inputs. In order to reconstruct the disrupted signals with high fidelity, the following penalty weightings are added. The following penalties are computed in the signal-space:

$$L_{dynamic\_range}(X_A) := |\max(x_A) - \max(x_{BA})|^2$$

$$L_{dynamic\_range}(X_B) := |\max(x_B) - \max(x_{AB})|^2$$

The learned weights for encoders $Enc_A$, $Enc_B$ and decoders $Dec_A$, $Dec_B$ are updated based on the backpropagation of generator losses and dynamic range loss at this stage.

Cycle consistency describes the ability to accurately reconstruct a single incoming signal by first transferring it across one domain, and then transferring the result across the other domain. In symbols, starting with a single disrupted signal and finishing with what should be a very similar signal, this appears as

$$X_B \xrightarrow{Enc_B} z_B \xrightarrow{Dec_A} x_{BA} \xrightarrow{Enc_A} z_{BA} \xrightarrow{Dec_B} x_{BAB}$$

As pointed out by UNIT, two cycle consistency measures can be made from a single input $x_B$ - one in the latent space: $d(z_B, z_{BA})$, and one in the signal space: $d(x_B, x_{BAB})$, where d is some distance metric. (Note: UNIT does not compare $z_B$ to $z_{BA}$ directly, rather they use the KL-divergence between a batch of latent vectors $\{z_B\}$ and the standard normal distribution, as well as the batch of latent vectors $\{z_{BA}\}$ and the standard normal distribution.) As a result, loss functions are applied to the latent space as well as in the signal space:

$$L_{cycle\_latent}(X_A) := MSE(z_A, z_{BA})$$

$$L_{cycle\_latent}(X_B) := MSE(z_B, z_{AB})$$

$$L_{cycle\_signal}(X_A) := L_1(x_A, x_{ABA})$$

$$L_{cycle\_signal}(X_B) := L_1(x_B, x_{BAB})$$

Learned weights are updated only for encoders $Enc_A$ and $Enc_B$ based on the backpropagation of these cycle losses at this stage.

The importance of cycle consistency in this context provides the opportunity to align the latent representation of non-disrupted signals with disrupted signals from the same class. Cycle consistency itself does not guarantee class alignment alone therefore a recovery drift penalty $L_{drift}$ is used to mitigate the undesirable class-swapping problem. Class swapping refers to the situation where the neural network will learn to accurately translate the set of signals in $X_B \rightarrow X_{BA}$, however the class identities are translated in a completely mismatched way. For example, a neural signal from a first class may be translated to a second class and vice versa. While this solution accurately reproduces the distribution of signals present in the original Dataset A, it leads to a completely dysfunctional BCI. One where, for example, the disrupted neural signal for moving one's index finger would be translated (and subsequently incorrectly decoded) as a clean signal indicating movement of the thumb.

To directly address the class swapping problem, deviations in the signal space between the input signal and the recovered (symmetrically the artificially disrupted) signal may be penalized. The intuition here is that disrupted signals are closer to non-disrupted signals of the same class than they are to non-disrupted signals of any other class. Therefore the loss functions for the signal-space penalties are applied during training:

$$L_{drift}(AB) := L_1(x_A, x_{AB})$$

$$L_{drift}(BA) := L_1(x_B, x_{BA})$$

The learned weights are updated for the encoders $Enc_A$, $Enc_B$ and decoders $Dec_A$, $Dec_B$ based on the backpropagation of these drift losses at this stage.

The autoencoder training models are updated according to how accurately the original signal $x_A$ and $X_B x B$ are reconstructed. Learned weights for the corresponding encoder and decoder are updated to optimize the reconstruction losses for each autoencoder model. The reconstruction losses are represented by

$$L_{AE}(Enc_A, Dec_A) := L_1(x_A, x_{AA})$$

$$L_{AE}(Enc_B, Dec_B) := L_1(x_B, x_{BB})$$

where $Enc_A$ and $Dec_A$ are updated based on reconstruction loss for autoencoder $L_{AE}(Enc_A, Dec_A)$, and similarly $Enc_B$ and $Dec_B$ are updated based on reconstruction loss for autoencoder $L_{AE}(Enc_B, Dec_B)$. A single epoch is achieved when the learned weights have been updated to accommodate all training objectives described above on all of the available data in Datasets A and B. The training proceeds through multiple epochs until the internal metric on the recovered set of signals reaches a predetermined value or relative minimum/maximum over a predetermined number of epochs. At this point, the translation model is considered to be trained. A typical translation model meets this threshold in 100 to 1000 epochs. The internal metric can be any measure which can be computed based on parameters or properties of the data or the neural network as long as it does not require ground-truth class labeling of the incoming disrupted signals. For example, as described above, it can be the difference between signal distributions of the clean $x_A$ and the recovered/translated set $X_{BA}$, or the entropy in decoded class values of the translated set $X_{BA}$. Whatever metric is used should retain a high correlation with the translated signal accuracy in order to be effective.

The strong correlation between high decoder entropy (for example) and high decoder accuracy, at the model level, enables both the selection of top performing models from among fully trained models, as well as the retrospective identification of optimal stopping points during training of single models to freeze neural network weights.

In an aspect, the strong correlation between high decoder entropy (for example) and high decoder, at the model level, enables both the selection of top performing models from among fully trained models, as well as the retrospective identification of optimal stopping points during training of single models to freeze neural network weights.

Applying Neural Signal Translation Models on Day$_{i+1}$

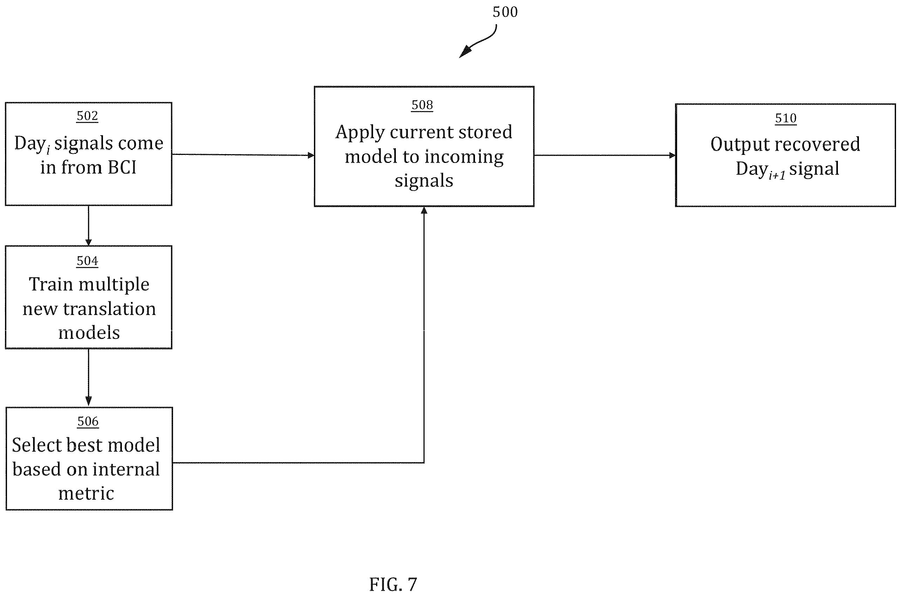

Each day a new model is trained and selected based on a new set of disrupted signals for that day. The signals disruption may vary on a daily basis making it necessary that the translation model is tailored to the specific disruption observed for that day. FIG. 7 illustrates an aspect of a translation model 500 where, a BCI receives 502 disrupted neural signals for day$_i$. The day$_i$ signals are stored in Dataset B and the ANNI method trains 504 multiple new translation model based on the day$_i$ disrupted signals. At this instance, a new translation model may not yet be selected for day$_{i+1}$. The ANNI method selects 506 the day$_{i+1}$ translation model, and the BCI applies 508 the selected translation model, trained on day$_i$ signals, for incoming signals on day$_{i+1}$. The BCI outputs 510 the recovered signals for the incoming signals received on day$_{i+1}$.

In another aspect, the incoming day$_{i+1}$ disrupted signals may comprises a partial dataset and the computing device may execute the ANNI method for an intraday translation mode. Once the intraday translation model is trained, the translation model trained on dayi signals is discontinued and immediately replaced with the intraday translation model. The intraday translation model is updated at the BCI and incoming neural signals may be recovered in real-time.

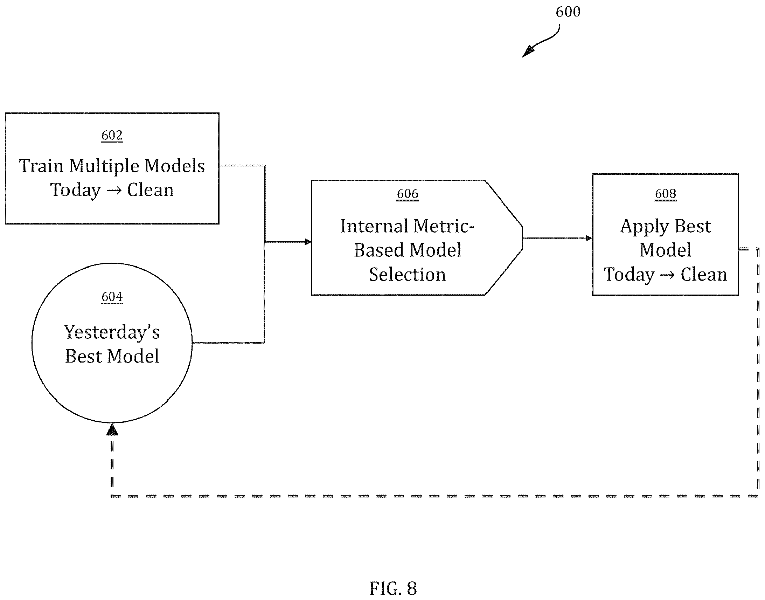

The translation model selected based on day$_i$ data may be very good at recovering data for day$_{i+1}$ and beyond. FIG. 8 illustrates a process 600 where the selected day$_i$ translation model is evaluated 604 against the trained 602 models for day$_{i+1}$. The internal metric value for the day$_i$ translation model is calculated and compared 606 with the values from the current day's batch of trained models. In this way, a single previously-trained 604 model may outperform all current trained 602 models and continue to be selected and applied 608 as the best performing model indefinitely.

Validation of the ANNI Method with Simulated Neural Signals

The accuracy of the ANNI method was validated by simulating neural signals from both MEA and ECoG interfaces. In the simulated validation process, the decoder accuracy was observed at over 90 % with all datasets with as much as 13 of 32 channels dropped. The MEA simulation model mimics the key components of neural responses recorded from an MEA interface. The model simulates a set of neurons with diverse stimulus responses making up a plurality of classes, where the class corresponds to the true stimulus identity. First, a response of a single neuron is determined by its response probability to a given stimulus, where the response probability is determined by heterogeneous Gaussian tuning curves (GTC). The mean and standard deviation (SD) values of the GTCs may be randomly selected within the stimulus range (for mean) or half the stimulus range (for SD). The GTCs then generate response probabilities for each neuron from stimuli values defined in time (400 ms with 1 ms sample rate). The stimuli in models may be static in time at 1 of 20 different values evenly distributed across the stimulus range. This process generates temporally constant response probabilities. Second, response probabilities may be used to generate spikes using a Poisson distribution to simulate noise in spike generation. The spikes counts may be binned (100 ms bins), generating 32x4 bin PSTHs (number of channels x number of time bins). Finally, a second additive noise source may be added to the PSTHs. The second additive noise has a SD of 10% of the PSTH mean and all resulting PSTH values below zero are rectified at zero. This process generated 5000 PSTH trials encoding 20 different stimulus classes.

The MEA disruption simulation models simulate signal degradation based on channel drop-out (drop) or neuron drop-in (swap) disruption models. In the channel drop-out model of neural disruption, each degradation step may be defined according to the loss of an entire electrode channel. Channel drop-out may be simulated by zeroing out all time bins in the PSTH for that particular channel in all samples. The channel drop-out loss is cumulative across the multiple degradation steps such that, half of the channels may contain only zeros. In contrast, a degradation step in the drop-in model may be defined according to a change in the stimulus response at an existing channel in the set. The stimulus response change may be modeled as a 10% positive or negative shift in the GTC mean (relative to the entire stimulus range) and an increase or decrease in the GTC SD of two times the original SD. Both models mimic common disruptions types observed in MEA sample recordings.

In contrast, the ECoG interface model simulates the LFP neural signals as they are observed at the electrodes rather than the underlying activity of neurons. The reason for this is that forward models of observed activity at the ECoG sensor derived from the underlying neuron populations will be specific to the neural subsystem being measured (e.g. somatosensory vs visual cortex) as well as the type and density of neural array. By simulating a more generic LFP signal and noise, the model sidesteps the implementation of more complex physical models required for simulating neuronal activity and point spread functions to approximate specific population responses while still replicating the fundamental signal qualities of ECoG reported in the literature (e.g. the frequency spectra, amplitude and noise characteristics).

The ECoG simulation model assumes that the basic time-domain response at any electrode (channel) will have an $S = 1/F^2$ distribution in the time-frequency domain, where S is the signal at a given channel and F is frequency in hertz. This spectral distribution closely approximates the characteristics of the ECoG interface. The model assumes that because ECoG samples are from a large cortical territory, the responses will have spatial heterogeneity. The model further assumes that changes in the observed ECoG signal that are relevant for decoding, typically manifest themselves as changes in lower frequency oscillations when averaged over multiple samples.

The LFP neural signals may be simulated on an 8x8 $\mu$ECoG array where a given stimulus or class may be instantiated with a series of signal inputs. Each signal input may require selecting a channel, frequency, phase, time (within the 1s signal window), or amplitude of a sinusoidal input. Finally, the spatial distance between electrodes in the array may be simulated with a unit grid (1mm spacing) and scaling the modulating input strength as a function of the distance from the center locus of the modulating input. Each modulating input may be added to the $1/F^2$ noise at the appropriate channels to produce a spatiotemporal pattern of activity that serves as the starting stimulus class template. Any of these parameters can be manipulated to investigate a different aspect of the model (i.e. array size, frequency of signal inputs etc.).

As described above, one of the primary sources of disruption for ECoG are changes in electrode impedance. These disruptions may be particularly egregious in the first 75 days after implant and may lead to dramatic changes in the variance and spectral power of signals. The disruption models use a linear model fit to simulate the degree the degree of changes in each spectral band of the signal over the first 100 days. The length of time since implant is used in the linear model to simulate the change in spectral power over time. The changes in the expected signals may then be computed by applying a discrete Fourier transform to each time-domain signal (e.g for each electrode channel and sample), applying a transform function to the signals in Fourier space with the appropriate frequency band coefficients from, and then using an inverse Fourier transform to restore the signals to the time domain. Beyond the first 100 days after implant, the interface may stabilize and the day-to-day or week-to-week changes in signal strength may be confined to a narrower operating range. The model may simulates this operating range as +/- 20% of the stable signal strength beyond day 100. These fluctuations may be randomly generated according to a Gaussian distribution centered at 0% signal change at each time step within the allowable bounds.

Another form of simulated disruption is cortical adaptation. Cortical adaptation refers to the change in the neural response itself, as the brain learns to more efficiently utilize the interface. The use of ECoG in chronic recordings and BCIs is limited currently and has restricted the study of adaptation to only a few studies. Those studies suggest that cortical adaptation manifests primarily as an increase in the amplitude of these signals over time. The simulated disruption model allows the signals to adapt continuously over the simulated lifetime of the implant in the following way. At each time point in the simulation (10 day steps), the model may allow 20% of the modulatory input signals to adapt, selected at random. Each time a signal adapts it can increase or decrease its amplitude by 10-20% prior to accounting for the impedance change. The reason for including a long-term depression (signal decrease) mechanism in our simulations was to prevent signal saturation over the longer simulation times (e.g. 360 days). The model may adapt signals at each time point (every 10 days for a total of 36) in the simulation and can be adapted multiple times over the time steps. The absolute magnitude of possible decrease or increase in a signal may be capped at 70 - 150% of its starting amplitude over the lifetime of the simulation. This results in a set of signals that continuously change their absolute and relative magnitude to each other over the lifetime of the simulation.

The simulation showed a rapid degradation in the first 100 days from calibration is the reason that many researchers do not train neural decoders until after the first 70-100 days, when the signals begin to stabilize. The disclosed aspect allows an implanted BCI to be trained immediately on $day_0$ datasets with high decoder accuracy.

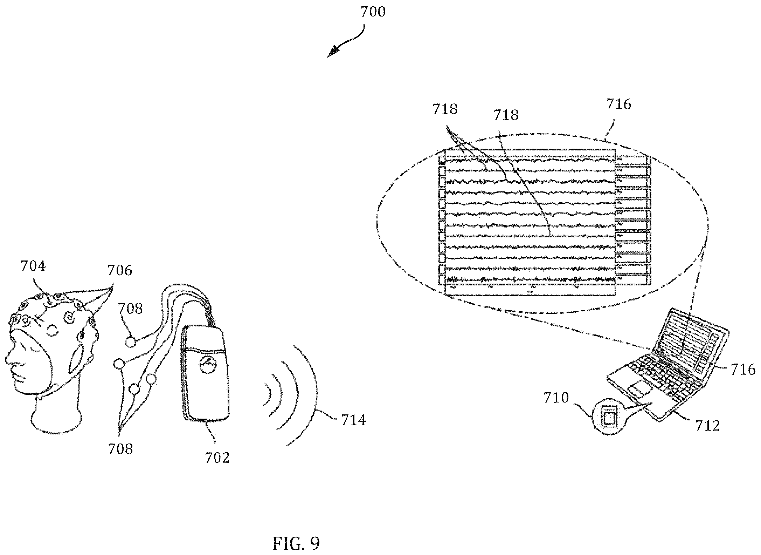

FIG. 9 illustrates one aspect of a neural signal stabilization system 700 comprising a wearable physiological signal amplifier system that records 32 channels of high-fidelity neural signals with water based electrodes 708, in accordance with at least one aspect of the present disclosure. The neural signal stabilization system 700 includes a BCI 702 with onboard 3D accelerometer and trigger channel, a configuration cap configured for 32-Channels, a head-cap 704 with 32 grommets 706 to receive the water based electrodes 708, water based electrodes 708, and ANNI method 710 that runs off a general purpose computer 712. The neural signal stabilization system 700 is configured to neural signals for a BCI. The neural signal stabilization system 700 can record many different signal types. In some aspects, the electrode configuration or signal type may be quickly changed by swapping out the configuration cap. The neural signal stabilization system 700 can telemeter 714 data back to the general purpose computer 712 running software 710 for real-time display 716 and analysis of the signals 718, or record it locally in a memory of the base unit 702 for later download to the computer 710. The neural signal stabilization system 700 can switch between WiFi mode, logger mode, or hybrid mode to suit a particular application protocol. The neural signal stabilization system is battery operated, rechargeable, and its compact size, integrated WiFi connectivity and flexibility are combined in a mobile physiologic measurement device.

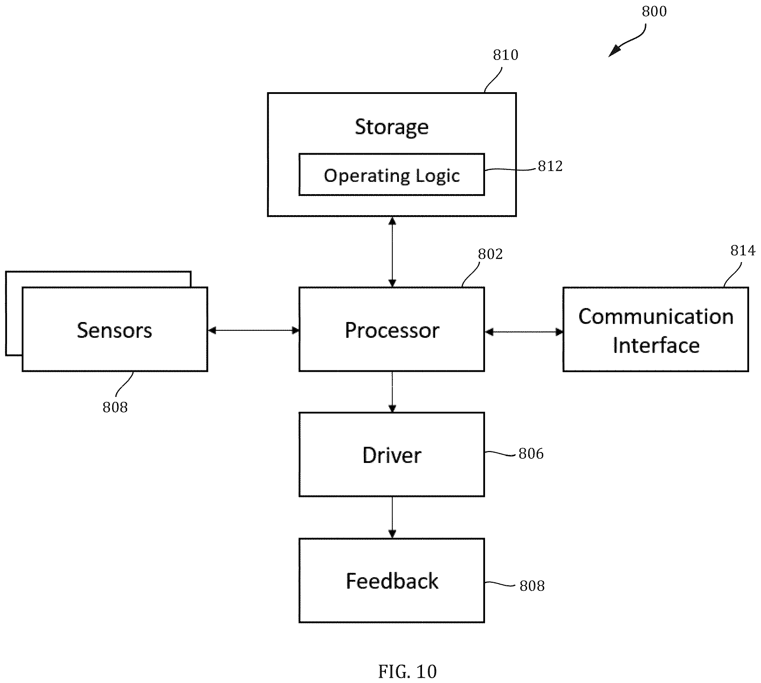

FIG. 10 illustrates an architectural or component view of a computing system 800 that may be employed to implement the ANNI method for training and selecting a neural signal translation model described in connection with FIGS. 1-8, in accordance with at least one aspect of the present disclosure. In various aspects, referring also to FIG. 9, the base unit 802 and/or the general purpose computer 712 may comprise the computing system 800 or portions of the computing system 800 to implement the ANNI method in accordance with the present disclosure. The computing system 800 may comprise one or more of the GPU processor 802 or the FPGA described previously. In various aspects, as illustrated, the computing system 800 comprises one or more processors 802 (e.g., microprocessor, microcontroller) coupled to various sensors 804 (e.g., neural electrodes) and at least one output feedback component 808 via a suitable circuit 806. For example, the output feedback component 808 may provide user intended response classification information to the BCI via a suitable circuit 806 such as a device driver 806. The one or more processors 802 may be any one of a number of single or multi-core processors known in the art. In addition, one or more storage devices 810 comprising operating logic 812 for operation is coupled to the one or more processors 802. The one or more storage devices 810 may include any suitable device such as a hard drive, random access memory (RAM), read only memory (ROM), or universal serial bus (USB) connected flash drives. The one or more storage device 810 may comprise volatile and non-volatile storage media configured to store persistent and temporal copies of the operating logic 812. The communication interface 814 may also be coupled to the one or more processors 802. In some aspects, one or more processors 802 may be packaged together with the operating logic 812 to, for example, form a System in Package (SiP) or a System on Chip (SoC). In other aspects, one or more processors 802 may be integrated on the same die with the operating logic 812.

In various aspects, the operating logic 812 may be implemented in instructions supported by the instruction set architecture (ISA) of one or more processors 802, or in higher level languages that are compiled into the supported ISA. The operating logic 812 may comprise one or more logic units or modules. In various aspects, the operating logic 812 is implemented in an object oriented manner. The operating logic 812 also can be configured to be executed in a multi-tasking and/or multi-thread manner. In other aspects, the operating logic 812 may be implemented in suitable hardware such as a gate array. In some aspects, the communication interface 814 may be configured to facilitate communication between a peripheral device and the computing system 800. The communication may include transmission of the collected neural signals, or brain waves to a hosting computer, and transmission of data associated therewith from the hosting computer to a peripheral device. In various aspects, the communication interface 814 may be a wired or a wireless communication interface. An example of a wired communication interface may include, but is not limited to, a USB interface. An example of a wireless communication interface may include, but is not limited to, a Bluetooth interface, WiFi, among others.

Having shown and described various aspects of the present disclosure, further adaptations of the methods and systems described herein may be accomplished by appropriate modifications by one of ordinary skill in the art without departing from the scope of the present disclosure. Several of such potential modifications have been mentioned, and others will be apparent to those skilled in the art. For instance, the examples, aspects, geometries, materials, dimensions, ratios, steps, and the like discussed above are illustrative and are not required. Accordingly, the scope of the present disclosure should be considered in terms of the following claims and is understood not to be limited to the details of structure and operation shown and described in the specification and drawings.

While various details have been set forth in the foregoing description, it will be appreciated that the various aspects of the method for neural signals stabilization may be practiced without these specific details. One skilled in the art will recognize that the herein described components (e.g., operations), devices, objects, and the discussion accompanying them are used as examples for the sake of conceptual clarity and that various configuration modifications are contemplated. Consequently, as used herein, the specific exemplars set forth and the accompanying discussion are intended to be representative of their more general classes. In general, use of any specific exemplar is intended to be representative of its class, and the non-inclusion of specific components (e.g., operations), devices, and objects should not be taken limiting.

Further, while several forms have been illustrated and described, it is not the intention of the applicant to restrict or limit the scope of the appended claims to such detail. Numerous modifications, variations, changes, substitutions, combinations, and equivalents to those forms may be implemented and will occur to those skilled in the art without departing from the scope of the present disclosure. Moreover, the structure of each element associated with the described forms can be alternatively described as a means for providing the function performed by the element. Also, where materials are disclosed for certain components, other materials may be used. It is therefore to be understood that the foregoing description and the appended claims are intended to cover all such modifications, combinations, and variations as falling within the scope of the disclosed forms. The appended claims are intended to cover all such modifications, variations, changes, substitutions, modifications, and equivalents.

For conciseness and clarity of disclosure, selected aspects of the foregoing disclosure have been shown in block diagram form rather than in detail. Some portions of the detailed descriptions provided herein may be presented in terms of instructions that operate on data that is stored in a computer memory. Such descriptions and representations are used by those skilled in the art to describe and convey the substance of their work to others skilled in the art. In general, an algorithm refers to a self-consistent sequence of steps leading to a desired result, where a "step" refers to a manipulation of physical quantities which may, though need not necessarily, take the form of electrical or magnetic signals capable of being stored, transferred, combined, compared, and otherwise manipulated. It is common usage to refer to these signals as bits, values, elements, symbols, characters, terms, numbers, or the like. These and similar terms may be associated with the appropriate physical quantities and are merely convenient labels applied to these quantities.

Unless specifically stated otherwise as apparent from the foregoing disclosure, it is appreciated that, throughout the foregoing disclosure, discussions using terms such as "processing" or "computing" or "calculating" or "determining" or "displaying" or the like, refer to the action and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

In a general sense, those skilled in the art will recognize that the various aspects described herein which can be implemented, individually and/or collectively, by a wide range of hardware, software, firmware, or any combination thereof can be viewed as being composed of various types of "electrical circuitry." Consequently, as used herein "electrical circuitry" includes, but is not limited to, electrical circuitry having at least one discrete electrical circuit, electrical circuitry having at least one integrated circuit, electrical circuitry having at least one application specific integrated circuit, electrical circuitry forming a general purpose computing device configured by a computer program (e.g., a general purpose computer configured by a computer program which at least partially carries out processes and/or devices described herein, or a microprocessor configured by a computer program which at least partially carries out processes and/or devices described herein), electrical circuitry forming a memory device (e.g., forms of random access memory), and/or electrical circuitry forming a communications device (e.g., a modem, communications switch, or optical-electrical equipment). Those having skill in the art will recognize that the subject matter described herein may be implemented in an analog or digital fashion or some combination thereof.

The foregoing detailed description has set forth various forms of the devices and/or processes via the use of block diagrams, flowcharts, and/or examples. Insofar as such block diagrams, flowcharts, and/or examples contain one or more functions and/or operations, it will be understood by those within the art that each function and/or operation within such block diagrams, flowcharts, or examples can be implemented, individually and/or collectively, by a wide range of hardware, software, firmware, or virtually any combination thereof. In one form, several portions of the subject matter described herein may be implemented via an application specific integrated circuits (ASIC), a field programmable gate array (FPGA), a digital signal processor (DSP), or other integrated formats. However, those skilled in the art will recognize that some aspects of the forms disclosed herein, in whole or in part, can be equivalently implemented in integrated circuits, as one or more computer programs running on one or more computers (e.g., as one or more programs running on one or more computer systems), as one or more programs running on one or more processors (e.g., as one or more programs running on one or more microprocessors), as firmware, or as virtually any combination thereof, and that designing the circuitry and/or writing the code for the software and or firmware would be well within the skill of one of skill in the art in light of this disclosure. In addition, those skilled in the art will appreciate that the mechanisms of the subject matter described herein are capable of being distributed as a program product in a variety of forms, and that an illustrative form of the subject matter described herein applies regardless of the particular type of signal bearing medium used to actually carry out the distribution. Examples of a signal bearing medium include, but are not limited to, the following: a recordable type medium such as a floppy disk, a hard disk drive, a Compact Disc (CD), a Digital Video Disk (DVD), a digital tape, a computer memory, etc.; and a transmission type medium such as a digital and/or an analog communication medium (e.g., a fiber optic cable, a waveguide, a wired communications link, a wireless communication link (e.g., transmitter, receiver, transmission logic, reception logic, etc.), etc.).

In some instances, one or more elements may be described using the expression "coupled" and "connected" along with their derivatives. It should be understood that these terms are not intended as synonyms for each other. For example, some aspects may be described using the term "connected" to indicate that two or more elements are in direct physical or electrical contact with each other. In another example, some aspects may be described using the term "coupled" to indicate that two or more elements are in direct physical or electrical contact. The term "coupled," however, also may mean that two or more elements are not in direct contact with each other, but yet still co-operate or interact with each other. It is to be understood that depicted architectures of different components contained within, or connected with, different other components are merely examples, and that in fact many other architectures may be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. Likewise, any two components so associated also can be viewed as being "operably connected," or "operably coupled," to each other to achieve the desired functionality, and any two components capable of being so associated also can be viewed as being "operably couplable," to each other to achieve the desired functionality. Specific examples of operably couplable include but are not limited to physically mateable and/or physically interacting components, and/or wirelessly interactable, and/or wirelessly interacting components, and/or logically interacting, and/or logically interactable components.

In other instances, one or more components may be referred to herein as "configured to," "configurable to," "operable/operative to," "adapted/adaptable," "able to," "conformable/conformed to," etc. Those skilled in the art will recognize that "configured to" can generally encompass active-state components and/or inactive-state components and/or standby-state components, unless context requires otherwise.

While particular aspects of the present disclosure have been shown and described, it will be apparent to those skilled in the art that, based upon the teachings herein, changes and modifications may be made without departing from the subject matter described herein and its broader aspects and, therefore, the appended claims are to encompass within their scope all such changes and modifications as are within the true scope of the subject matter described herein. It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to claims containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations.

In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations).

Furthermore, in those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that typically a disjunctive word and/or phrase presenting two or more alternative terms, whether in the description, claims, or drawings, should be understood to contemplate the possibilities of including one of the terms, either of the terms, or both terms unless context dictates otherwise. For example, the phrase "A or B" will be typically understood to include the possibilities of "A" or "B" or "A and B."

With respect to the appended claims, those skilled in the art will appreciate that recited operations therein may generally be performed in any order. Also, although various operational flows are presented in a sequence(s), it should be understood that the various operations may be performed in other orders than those which are illustrated, or may be performed concurrently. Examples of such alternate orderings may include overlapping, interleaved, interrupted, reordered, incremental, preparatory, supplemental, simultaneous, reverse, or other variant orderings, unless context dictates otherwise. Furthermore, terms like "responsive to," "related to," or other past-tense adjectives are generally not intended to exclude such variants, unless context dictates otherwise.

It is worthy to note that any reference to "one aspect," "an aspect," "one form," or "a form" means that a particular feature, structure, or characteristic described in connection with the aspect is included in at least one aspect. Thus, appearances of the phrases "in one aspect," "in an aspect," "in one form," or "in an form" in various places throughout the specification are not necessarily all referring to the same aspect. Furthermore, the particular features, structures or characteristics may be combined in any suitable manner in one or more aspects.

With respect to the use of substantially any plural and/or singular terms herein, those having skill in the art can translate from the plural to the singular and/or from the singular to the plural as is appropriate to the context and/or application. The various singular/plural permutations are not expressly set forth herein for sake of clarity.

In certain cases, use of a system or method may occur in a territory even if components are located outside the territory. For example, in a distributed computing context, use of a distributed computing system may occur in a territory even though parts of the system may be located outside of the territory (e.g., relay, server, processor, signal-bearing medium, transmitting computer, receiving computer, etc. located outside the territory).

A sale of a system or method may likewise occur in a territory even if components of the system or method are located and/or used outside the territory. Further, implementation of at least part of a system for performing a method in one territory does not preclude use of the system in another territory.

All of the above-mentioned U.S. patents, U.S. patent application publications, U.S. patent applications, foreign patents, foreign patent applications, non-patent publications referred to in this specification and/or listed in any Application Data Sheet, or any other disclosure material are incorporated herein by reference, to the extent not inconsistent herewith. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

In summary, numerous benefits have been described which result from employing the concepts described herein. The foregoing description of the one or more forms has been presented for purposes of illustration and description. It is not intended to be exhaustive or limiting to the precise form disclosed. Modifications or variations are possible in light of the above teachings. The one or more forms were chosen and described in order to illustrate principles and practical application to thereby enable one of ordinary skill in the art to utilize the various forms and with various modifications as are suited to the particular use contemplated. It is intended that the claims submitted herewith define the overall scope.

Additional aspects of methods for neural signals stabilization are described in Generative Adversarial Neural Networks Maintain Decoder Accuracy During Signal Disruption In Simulated Long-term Recordings From Brain Computer Interfaces, by Thomas Stephens, Jon Cafaro, Ryan MacRae, Stephen Simons, which is incorporated herein by reference and appended hereto as Appendix A.

Various aspects of the subject matter described herein are set out in the following examples.

Example 1: A method for translating disrupted neural signals comprising: receiving, at a brain-computer interface (BCI), a first set of neural signals and a second set of neural signals, wherein the first set of neural signals comprises $day_0$ data and wherein the second set of neural signals comprises $day_i$ data; transmitting, to a computing device comprising at least one processor coupled to at least one memory unit, the first set of neural signals and the second set of neural signals and storing the signals in a first datastore and a second datastore of the computing device; training, by the computing device, a plurality of neural translation models in an adversarial networks for neural interfaces (ANNI) method, wherein the ANNI method trains a plurality of translation models, and wherein the plurality of translation models comprise a first autoencoder model, a second autoencoder model, a disrupted recovery model, an artificial disruption model, a first discriminator model, a second discriminator model, shared latent space model, a shared signal space model, and a penalty drift model with training objectives that achieve a shared latent space which retains signal class information, and penalize signal class swapping; generating, by the computing device, a model loss function for each of the plurality of the neural translation models; deriving, by the computing device, a weighting value for each of the plurality of neural translation models, wherein the weighting value corresponds to the loss function for each of the plurality of neural translation models; calculating, by the computing device, an internal metric value for each epoch; determining, by the computing device, whether the internal metric value meets a predetermined value or meet a predetermined number of epochs; updating, by the computing device, the weighting values for the plurality of neural translation models when the predetermined internal metric value is not met; and translating, by the BCI, a plurality of $day_{i+1}$ incoming signals based on the selected model from the $day_i$ signals, wherein today is $day_{i+1}$; and wherein a $day_{i+1}$ translation model is not yet selected; selecting, by the computing device, the $day_i$ translation model based on the weighting values of the plurality of neural translation models that correspond to the epoch with the highest internal metric value; and translating, by the BCI, the $day_{i+1}$ incoming neural signals with the $day_i$ translation model.

Example 2: The method for stabilizing neural signals of Example 1, wherein the $day_0$ data comprises data received the same day that the BCI is calibrated by a clinician, and wherein the $day_0$ data corresponds to undisrupted neural signals.

Example 3: The method for stabilizing neural signals of any one or more of Examples 1-2, wherein the first set of neural signals in the first datastore is unpaired to the second set of neural signals in the second datastore.

Example 4: The method for stabilizing neural signals of any one or more of Examples 1-3, wherein the plurality of $day_{i+1}$ incoming neural signals are translated with the $day_i$ translation model, and the computing device is training a new $day_{i+1}$ translation model based on a partial dataset of the plurality of $day_{i+1}$ incoming neural signals, and wherein the $day_i$ translation model is immediately replaced with the $day_{i+1}$ translation model once the new $day_{i+1}$ translation model is trained and selected.

Example 5: The method for stabilizing neural signals of any one or more of Examples 1-4, wherein the BCI and the computing device are integrated into the same device.

Example 6: The method for stabilizing neural signals of any one or more of Examples 1-5, wherein the BCI and computing device send and receive signals over a wireless communication interface.

Example 7: The method for stabilizing neural signals of any or more of Examples 1-6, wherein the internal metric for determining training completion and model selection is the difference between signal distributions according to the mean and variance of the translated signals and the $day_0$ signals.

Example 8: The method for stabilizing neural signals of any one or more of Examples 1-7, wherein the internal metric for determining training completion and model selection is the entropy of decoded class values across translated signals and based on the BCI's original $day_0$ decoder.

Example 9: The method for stabilizing neural signals of any one or more of Examples 1-8, wherein the cycle-consistency penalty is applied in the latent space as a direct comparison between the clean and disrupted latent representations of the same signal in order to separate the shared latent space by signal class.

Example 10: The method for stabilizing neural signals of any one or more of Examples 1-9, wherein a class drift penalty is applied such that recovered signals are required to resemble their original disrupted version in order to deter the problem of class swap.

Example 11: A method for translating disrupted neural signals comprising: receiving, at a brain-computer interface (BCI), a first set of neural signals and a second set of neural signals, wherein the first set of neural signals comprises $day_0$ data and wherein the second set of neural signals comprises $day_i$ data; transmitting, to a computing device comprising at least one processor coupled to at least one memory unit, the first set of neural signals and the second set of neural signals and storing the signals in a first datastore and a second datastore of the computing device; training, by the computing device, a plurality of neural translation models in an adversarial networks for neural interfaces (ANNI) method, wherein the ANNI method trains a plurality of translation models, and wherein the plurality of translation models comprise a first autoencoder model, a second autoencoder model, a disrupted recovery model, an artificial disruption model, a first discriminator model, a second discriminator model, shared latent space model, a shared signal space model, and a penalty drift model with training objectives that achieve a shared latent space which retains signal class information, and penalize signal class swapping; generating, by the computing device, a model loss function for each of the plurality of the neural translation models; deriving, by the computing device, a weighting value for each of the plurality of neural translation models, wherein the weighting value corresponds to the loss function for each of the plurality of neural translation models; calculating, by the computing device, an internal metric value for each epoch; determining, by the computing device, whether the internal metric value meets a predetermined value or meet a predetermined number of epochs; updating, by the computing device, the weighting values for the plurality of neural translation models when the predetermined internal metric value is not met; and translating, by the BCI, a plurality of $day_{i+1}$ incoming signals based on the selected model from the $day_i$ signals, wherein today is $day_{i+1}$; and wherein a $day_{i+1}$ translation model is not yet selected; selecting, by the computing device, the $day_i$ translation model based on the weighting values of the plurality of neural translation models that correspond to the epoch with the highest internal metric value; and translating, by the BCI, the $day_{i+1}$ incoming neural signals with the $day_i$ translation model.

Example 12: The non-transitory computer readable storage medium of Example 11, wherein the $day_0$ data comprises data received the same day that the BCI is calibrated by a clinician, and wherein the $day_0$ data corresponds to undisrupted neural signals.

Example 13: The non-transitory computer readable storage medium of any one or more of Examples 11-12, wherein the first set of neural signals in the first datastore is unpaired to the second set of neural signals in the second datastore.

Example 14: The non-transitory computer readable storage medium of any one or more of Examples 11-13, wherein the plurality of $day_{i+1}$ incoming neural signals are translated with the $day_i$ translation model, and the computing device is training a new $day_{i+1}$ translation model based on a partial dataset of the plurality of $day_{i+1}$ incoming neural signals, and wherein the $day_i$ translation model is immediately replaced with the $day_{i+1}$ translation model once the new $day_{i+1}$ translation model is trained and selected

## Claims

1. A method for translating disrupted neural signals comprising:

    receiving, at a brain-computer interface (BCI), a first set of neural signals and a second set of neural signals, wherein the first set of neural signals comprises $day_0$ data and wherein the second set of neural signals comprises $day_i$ data;
    transmitting, to a computing device comprising at least one processor coupled to at least one memory unit, the first set of neural signals and the second set of neural signals and storing the signals in a first datastore and a second datastore of the computing device;
    training, by the computing device, a plurality of neural translation models in an adversarial networks for neural interfaces (ANNI) method, wherein the ANNI method trains a plurality of translation models, and wherein the plurality of translation models comprise a first autoencoder model, a second autoencoder model, a disrupted recovery model, an artificial disruption model, a first discriminator model, a second discriminator model, shared latent space model, a shared signal space model, and a penalty drift model with training objectives that achieve a shared latent space which retains signal class information, and penalize signal class swapping;
    generating, by the computing device, a model loss function for each of the plurality of the neural translation models;
    deriving, by the computing device, a weighting value for each of the plurality of neural translation models, wherein the weighting value corresponds to the loss function for each of the plurality of neural translation models;
    calculating, by the computing device, an internal metric value for each epoch;
    determining, by the computing device, whether the internal metric value meets a predetermined value or meet a predetermined number of epochs;
    updating, by the computing device, the weighting values for the plurality of neural translation models when the predetermined internal metric value is not met; and
    translating, by the BCI, a plurality of $day_{i+1}$ incoming signals based on the selected model from the $day_i$ signals, wherein today is $day_{i+1}$; and wherein a $day_{i+1}$ translation model is not yet selected;
    selecting, by the computing device, the $day_i$ translation model based on the weighting values of the plurality of neural translation models that correspond to the epoch with the highest internal metric value; and
    translating, by the BCI, the $day_{i+1}$ incoming neural signals with the $day_i$ translation model.

2. The method for stabilizing neural signals of claim 1, wherein the $day_0$ data comprises data received the same day that the BCI is calibrated by a clinician, and wherein the $day_0$ data corresponds to undisrupted neural signals.

3. The method for stabilizing neural signals of claim 1, wherein the first set of neural signals in the first datastore is unpaired to the second set of neural signals in the second datastore.

4. The method for stabilizing neural signals of claim 1, wherein the plurality of $day_{i+1}$ incoming neural signals are translated with the $day_i$ translation model, and the computing device is training a new $day_{i+1}$ translation model based on a partial dataset of the plurality of $day_{i+1}$ incoming neural signals, and wherein the $day_i$ translation model is immediately replaced with the $day_{i+1}$ translation model once the new $day_{i+1}$ translation model is trained and selected.

5. The method for stabilizing neural signals of claim 1, wherein the BCI and the computing device are integrated into the same device.

6. The method for stabilizing neural signals of claim 1, wherein the BCI and computing device send and receive signals over a wireless communication interface.

7. The method for stabilizing neural signals of claim 1, wherein the internal metric for determining training completion and model selection is the difference between signal distributions according to the mean and variance of the translated signals and the $day_0$ signals.

8. The method for stabilizing neural signals of claim 1, wherein the internal metric for determining training completion and model selection is the entropy of decoded class values across translated signals and based on the BCI's original $day_0$ decoder.

9. The method for stabilizing neural signals of claim 1, wherein the cycle-consistency penalty is applied in the latent space as a direct comparison between the clean and disrupted latent representations of the same signal in order to separate the shared latent space by signal class.

10. The method for stabilizing neural signals of claim 1, wherein a class drift penalty is applied such that recovered signals are required to resemble their original disrupted version in order to deter the problem of class swap.

11. A non-transitory computer readable storage medium comprising instructions stored thereon, when executed by one or more processors coupled to one or more memory units, perform operations comprising:

receiving, a first set of neural signals and a second set of neural signals from a BCI, wherein the first set of neural signals comprises $day_0$ data and wherein the second set of neural signals comprises $day_i$ data;
transmitting, the first set of neural signals and the second set of neural signals and storing the signals in a first datastore and a second datastore of the computing device;
training, a plurality of neural translation models in an adversarial networks for neural interfaces (ANNI) method, wherein the ANNI method trains a plurality of translation models, and wherein the plurality of translation models comprise a first autoencoder model, a second autoencoder model, a disrupted recovery model, an artificial disruption model, a first discriminator model, a second discriminator model, shared latent space model, a shared signal space model, and a penalty drift model;
generating, a model loss function for each of the plurality of the neural translation models;
deriving, a weighting value for each of the plurality of neural translation models, wherein the weighting value corresponds to the loss function for each of the plurality of neural translation models;
calculating, an internal metric value for each epoch;
determining, whether the internal metric value meets a predetermined value or meet a predetermined number of epochs;
updating, the weighting values for the plurality of neural translation models when the predetermined internal metric value is not met; and
translating, a plurality of $day_{i+1}$ incoming signals based on the selected model from the $day_i$ signals, wherein today is $day_{i+1}$; and wherein a $day_{i+1}$ translation model is not yet selected;
selecting, the $day_i$ translation model based on the weighting values of the plurality of neural translation models that correspond to the epoch with the highest internal metric value; and
translating, the $day_{i+1}$ incoming neural signals with the $day_i$ translation model.

12. The non-transitory computer readable storage medium of Claim 11, wherein the $day_0$ data comprises data received the same day that the BCI is calibrated by a clinician, and wherein the $day_0$ data corresponds to undisrupted neural signals.

13. The non-transitory computer readable storage medium of Claim 11, wherein the first set of neural signals in the first datastore is unpaired to the second set of neural signals in the second datastore.

**14.** The non-transitory computer readable storage medium of Claim 11, wherein the plurality of $day_{i+1}$ incoming neural signals are translated with the $day_i$ translation model, and the computing device is training a new $day_{i+1}$ translation model based on a partial dataset of the plurality of $day_{i+1}$ incoming neural signals, and wherein the $day_i$ translation model is immediately replaced with the $day_{i+1}$ translation model once the new $day_{i+1}$ translation model is trained and selected.

```
                    ┌─────────────────────────────────────────┐
              2 ───│      Receive Day₀ and Dayᵢ data          │◄─────┐
                    └─────────────────────────────────────────┘      │
                                      │                              │
                                      ▼                              │
                    ┌─────────────────────────────────────────┐      │
              4 ───│       Train a plurality of models with   │      │
                    │       Day₀ data and Dayᵢ data            │      │
                    └─────────────────────────────────────────┘      │
                                      │                              │
                                      ▼                              │
                    ┌─────────────────────────────────────────┐      │
              6 ───│  Determine training is completed for models│     │
                    │ based on internal metric (e.g. decoder entropy)│ │
                    └─────────────────────────────────────────┘      │
                                      │                              │
                                      ▼                              │
                    ┌─────────────────────────────────────────┐      │
              8 ───│  Select model using internal metric (e.g. model│ │
                    │        with highest decoder entropy)     │      │
                    └─────────────────────────────────────────┘      │
                                      │                              │
                                      ▼                              │
                    ┌─────────────────────────────────────────┐      │
             10 ───│    Translate incoming Dayᵢ signals with  │      │
                    │             selected model               │      │
                    └─────────────────────────────────────────┘      │
                                      │                              │
                                      ▼                              │
                    ┌─────────────────────────────────────────┐      │
             12 ───│   Repeat training and selection process for│─────┘
                    │       subsequent day signals, i=i+1      │
                    └─────────────────────────────────────────┘
```

FIG. 1

FIG. 2

200

| |
|---|
| Store $Day_0$ data and $Day_i$ data in datastores A and B respectively |

202

| |
|---|
| Select samples $x_A$ and $x_B$ from datastores A and B respectively |

204

| |
|---|
| Map $x_A$ into the shared latent space Z via $Enc_A(x_A)$ |

206

| |
|---|
| Map $x_B$ into the shared latent space Z via $Enc_B(x_B)$ |

208

| |
|---|
| Reconstruct $x_{AA} = Dec_A(Enc_A(x_A))$ and $x_{BA} = Dec_A(Enc_B(x_B))$ |

210

| |
|---|
| Reconstruct $x_{BB} = Dec_B(Enc_B(x_B))$ and $x_{AB} = Dec_B(Enc_A(x_A))$ |

212

FIG. 3

FIG. 4

FIG. 5

FIG. 6

FIG. 7

600

**602**

Train Multiple Models
Today → Clean

**604**

Yesterday's
Best Model

**606**

Internal Metric-
Based Model
Selection

**608**

Apply Best
Model
Today → Clean

FIG. 8

FIG. 9

EP 4 092 580 A1

FIG. 10

**EP 4 092 580 A1**

Europäisches
Patentamt

European
Patent Office

Office européen
des brevets

# EUROPEAN SEARCH REPORT

Application Number

EP 22 17 3023

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ALI FARSHCHIAN ET AL: "Adversarial Domain Adaptation for Stable Brain-Machine Interfaces", ARXIV.ORG, CORNELL UNIVERSITY LIBRARY, 201 OLIN LIBRARY CORNELL UNIVERSITY ITHACA, NY 14853, 28 September 2018 (2018-09-28), XP081017006, * abstract; Sections 1 and 2-6; figures 1,3,4 * | 1-14 | INV.<br>G06N3/04<br>G06N3/08<br>G06F3/01 |
| A | Shriki Oren ET AL: "EEGNAS : Neural Architecture Search for Electroencephalography Data Analysis and Decoding", , October 2019 (2019-10), pages 1-18, XP055970646, Retrieved from the Internet: URL:https://www.researchgate.net/profile/E lad-Rapaport-2/publication/336375192_EEGNA S_Neural_Architecture_Search_for_Electroen cephalography_Data_Analysis_and_Decoding/l inks/5d9f09eb299bf13f40d1713f/EEGNAS-Neura l-Architecture-Search-for-Electroencephalo graphy-Data-Analysis-and-Decoding.pdf [retrieved on 2022-10-12] * abstract; Sections 1 and 3-4 * | 1-14 | |

-/--

TECHNICAL FIELDS SEARCHED (IPC)

G06N
G06F
A61B

The present search report has been drawn up for all claims

| Place of search | Date of completion of the search | Examiner |
|---|---|---|
| The Hague | 13 October 2022 | Klasen, TJ |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

28

Europäisches
Patentamt

European
Patent Office

Office européen
des brevets

## EUROPEAN SEARCH REPORT

**Application Number**

EP 22 17 3023

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GANDHI SUNIL ET AL: "Denoising Time Series Data Using Asymmetric Generative Adversarial Networks", 17 June 2018 (2018-06-17), SAT 2015 18TH INTERNATIONAL CONFERENCE, AUSTIN, TX, USA, SEPTEMBER 24-27, 2015; [LECTURE NOTES IN COMPUTER SCIENCE; LECT.NOTES COMPUTER], SPRINGER, BERLIN, HEIDELBERG, PAGE(S) 285 - 296, XP047475733, ISBN: 978-3-540-74549-5 [retrieved on 2018-06-17] * abstract; Sections 1 and 3-5; figures 1,2 * | 1-14 | |
| A | YAO YUE ET AL: "A Feature Filter for EEG Using Cycle-GAN Structure", 18 November 2018 (2018-11-18), ADVANCES IN DATABASES AND INFORMATION SYSTEMS; [LECTURE NOTES IN COMPUTER SCIENCE; LECT.NOTES COMPUTER], SPRINGER INTERNATIONAL PUBLISHING, CHAM, PAGE(S) 567 - 576, XP047496645, ISBN: 978-3-319-10403-4 [retrieved on 2018-11-18] * abstract; Sections 1 and 3-6; figure 4 * | 1-14 | TECHNICAL FIELDS SEARCHED (IPC) |
| A | YOUNG-EUN LEE ET AL: "Reconstructing ERP Signals Using Generative Adversarial Networks for Mobile Brain-Machine Interface", ARXIV.ORG, CORNELL UNIVERSITY LIBRARY, 201 OLIN LIBRARY CORNELL UNIVERSITY ITHACA, NY 14853, 18 May 2020 (2020-05-18), XP081675174, * abstract; Sections I-IV; figures 2, 3 * | 1-14 | |

The present search report has been drawn up for all claims

| Place of search | Date of completion of the search | Examiner |
|---|---|---|
| The Hague | 13 October 2022 | Klasen, TJ |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**EP 4 092 580 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63188085
- US 63189527
- US 16044221
- US 35402919
- US 72062117
- US 10660569 B
- US 67884817
- US 10945864 B